# EUROPEAN PATENT SPECIFICATION

(11) **EP 1 115 373 B1**
(45) Date of publication and mention of the grant of the patent: **22.12.2004**
(21) Application number: 99953749.1
(22) Date of filing: 20.09.1999
(51) Int. Cl.: A61K 7/20, A61K 7/16

(54) **ORAL COMPOSITION WITH AN IMPROVED TEETH WHITENING EFFECT**
ORALE ZUSAMMENSETZUNG MIT VERBESSERTEM BLEICHENDEM EFFEKT
COMPOSITION D'HYGIENE BUCCALE DOTEE D'UN POUVOIR ACCRU DE BLANCHIMENT DES DENTS

(30) Priority: 25.09.1998 EP 98307733
(43) Date of publication of application: 18.07.2001
(73) Proprietor: UNILEVER N.V., 3013 AL Rotterdam (NL); UNILEVER PLC, London EC4P 4BQ (GB)
(72) Inventor: JOINER, Andrew, Unilever Research Port Sunlight, Bebington, Merseyside L63 3JW (GB); THORNTHWAITE, David William, Unilever Research, Bebington, MerseysideL63 3JW (GB)
(74) Representative: Kan, Jacob Hendrik
(86) International application number: PCT/EP1999/007379
(87) International publication number: WO 2000/018366

(56) References cited:
- EP-A- 0 424 020
- WO-A-96/05802
- US-A- 5 360 568
- US-A- 5 785 886

## Description

The present invention relates to an oral composition with an improved teeth whitening effect as defined in claims 1 to 3. More particularly, it relates to an oral composition with an improved teeth whitening effect comprising a safe and effective amount of certain organic peroxy compounds and certain catalysts, which are capable of reacting with the organic peroxy compounds to generate more reactive oxygen species.

The use of peroxy compounds in oral care compostions has already been proposed in the prior art. Many peroxy compounds have been suggested for whitening/bleaching human teeth, and representative examples of such peroxy compounds are hydrogen peroxide, urea peroxide, organic peracids such as perphthalic acid, diperoxycarboxylic acids, 1,12-dodecanedioic peroxy acid, peroxy acetic acid and systems comprising a peroxy compound and a peroxy acid precursor which generate peroxy acetic acid in situ, such as sodium perborate and tetraacetylethylene diamine (TAED). The use of peroxy acetic acid is suggested in particular in e.g. EP-A-0545,594 (Colgate), which also sets out the various prior proposals, made in the art for several peroxy compounds as bleaching/whitening agent for human teeth.

In our WO-A-96/05802 we have described the use of various organic peroxyacids as teethwhitening agents.

We have now found that certain organic peroxy compounds, which will be defined hereinafter, when used together with certain catalysts, defined hereinafter, are much more effective than the organic peroxy compounds above, even in the absence of a peroxy acid precursor (bleach precursor), producing a teethwhitening effect much more rapidly. These certain organic peroxy compounds are selected from the group consisting of:
1) peroxy amido phthalamides having the structural formula: 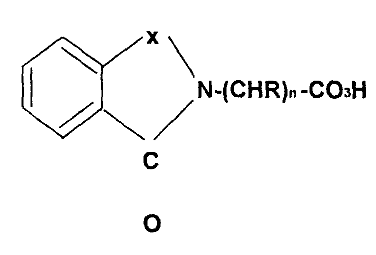 in which R = hydrogen or C₁-C₄ alkyl;
   n = 1 to 5; and
   X = C=0 or SO₂
2) sulphoperbenzoic acid ("SPB"),
3) monoperoxyphthalic acid ("MPP"), and
4) (per)acetylperoxyboric acid
and/or salts thereof.

The peroxy amido phthalamides of formula 1) are known per se and have been described in EP-A-325,288 and EP-A-325,289. A preferred compound of this formula is N-phthalimido hexanoic peroxy acid ("PAP") of formula 1), in which R = H, n = 5 and X = C=0. An example of a compound according to formula 1) wherein X = SO₂ is saccharinperhexanoic acid ("saccharin PAP"), as described in EP-A-485,927. The salts of these peroxyacids are preferably the alkalimetal salts.

The salts of sulphoperbenzoic are preferably the alkalimetal salts, particularly preferably the potassium salt ("KSPB"). This peroxyacid has been described in EP-A-124,968 and EP-A-212,913.

The salts of monoperoxyphthalic acid are preferably the alkalimetal and alkaline earth metal salts, particularly preferably the magnesium salt. The use of magnesium monoperoxyphthalate in oral care products has been described in US-A-4,670,252.

(Per)acetylperoxyboric acid and salts thereof are also known per se from EP-A-212,913.

Preferably, the peroxy compound is PAP and/or KSPB.

The amount of the peroxy compounds,used in the present invention, may vary from 0.01 to 99 % by weight of the oral composition, preferably from 0.1 to 30 % by weight, particularly preferably from 0.1-5 % by weight.

The catalysts, used in the present invention are imine quaternary salts as described in US-A-5,360,568 and US-A-5,360,569. Suitable examples of these imine quaternary salts are of the formula: 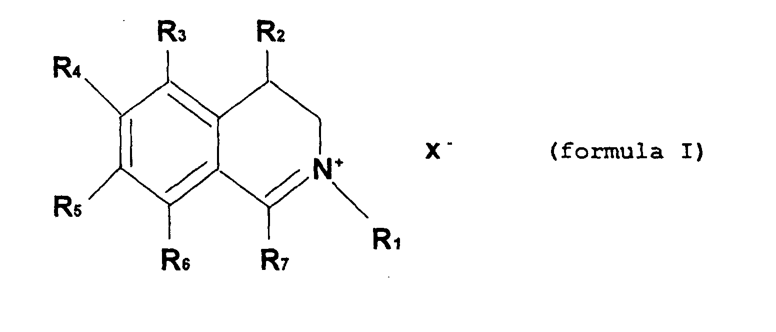 wherein R₁ is hydrogen or a C₁-C₈ alkyl group, R₂ is hydrogen, or a phenyl group or a keto group, R₃, R₄ and R₅ are hydrogen or -O-R¹, whereby R¹ is a C₁-C₄ alkyl group, R₆ and R₇ are hydrogen or a C₁-C₄ alkyl group and X is a counterion, stable in the presence of oxidizing agents, comprising Br⁻, BF₄⁻, Cl⁻, CH₃SO₄⁻, tosylate⁻, PF₆⁻, F⁻, fluorophosphate cations and C₁₂-alkylsulphate cations.

A preferred catalyst is an imine quaternary salt of the above formula, in which R₁ = methyl, and R₂-R₆ are all hydrogen, and X is tosylate ("Imine Quat 200").

The catalyst is used generally in an amount of 0.01 to 5 % by weight, preferably 0.05 to 1.5 % by weight, and particulary preferably 0.1 to 1 % by weight of the oral care composition.

The oral compositions can be formulated in any suitable application form, such as gels, mouthwashes, toothpowders and toothpastes. They may be formulated into a single formulation or they may be formulated for multi compartment containers into different formulations, e.g. one containing the peroxy compound and ingredients compatible therewith, and another containing the remaining ingredients.

The oral care compositions of the present invention may furthermore comprise optional, conventional ingredients such as pharmaceutically acceptable carriers like starch, sucrose, water or water/alcohol systems etc.. Small amounts of surfactants may also be included, such as anionic, nonionic and amphoteric surfactants. When formulated into a dentifrice, such formulation may contain all the usual dentifrice ingredients.

Thus, they may comprise particulate abrasive materials such as silicas, aluminas, calcium carbonates, dicalciumphosphates, calcium pyrophosphates hydroxyapatites, trimetaphosphates, insoluble hexametaphosphates, agglomerated particulate abrasive materials and so on, usually in amounts between 5 and 60 % by weight.

Furthermore, the dentifrice formulations may comprise humectants such as glycerol, sorbitol, propyleneglycol, xylitol, lactitol and so on.

Binders and thickeners such as sodium carboxymethylcellulose, xanthan gum, gum arabic etc. may also be included, as well as synthetic polymers such as polyacrylates and carboxyvinyl polymers such as Carbopol®.

Flavours such as peppermint and spearmint oils may also be included, as well as preservatives, opacifying agents, colouring agents, pH-adjusting agents, sweetening agents and so on. Stabilising agents for the organic peroxy compounds such as dipicolinic acid or sodium stannate may also be usefully included.

Anti-bacterial agents may also be included such as Triclosan, chlorhexidine, copper-, zinc- and stannous salts such as zinc citrate, sodium zinc citrate and stannous pyrophosphate, sanguinarine extract, metronidazole. Further examples of anti-bacterial agents are quaternary ammonium compounds such as cetylpyridinium chloride; bis-guanides such as chlorhexidine digluconate, hexetidine, octenidine, alexidine; halogenated bisphenolic compounds such as 2,2' methylenebis-(4-chloro-6-bromophenol).

Polymeric compounds which can enhance the delivery of active ingredients such as anti-bacterial agents can also be included. Examples of such polymers are copolymers of polyvinylmethylether with maleic anhydride and other similar delivery enhancing polymers, e.g. those described in DE-A-3,942,643 (Colgate)

Furthermore anti-inflammatory agents such as ibuprofen, flurbiprofen, aspirin, indomethacin etc. may also be included.

Anti-caries agents such as sodium- and stannous fluoride, aminefluorides, monosodiumfluorophosphate, casein, plaque buffers such as urea, calcium lactate, calcium glycerophosphate, strontium polyacrylates may also be included. Other optional ingredients include vitamins such as Vitamin C, and plant extracts. Desensitising agents such as potassium citrate, potassium chloride, potasium tartrate, potassium bicarbonate, potassium oxalate, potassium nitrate as well as strontium salts may also be included.

Buffers and salts to buffer the pH and ionic strength of the compositions may also be included. The pH of the compositions usually ranges from 5-10, preferably 6-9 and especially preferably 7-8.5.

Liposomes and other encapsulates may also be used to improve delivery or stability of active ingredients.

Furthermore, the oral compositions may comprise anti-calculus agents such as alkalimetal pyrophosphates, hypophosphite-containing polymers, organic phosphonates, phosphocitrates etc..

In addition, the compositions may comprise functional biomolecules such as bacteriocins, antibodies, enzymes and so on.

Other optional ingredients that may be included are e.g. effervescing systems such as sodium bicarbonate/citric acid systems, colour change systems, and so on.

Preferably, the compositions do not contain a bleach precursor.

When formulated as a mouthwash, the oral care composition usually comprises a water/alcohol solution, flavour, humectant, sweetener and colorant.

Since the peroxy compounds of the invention also have an anti-microbial property, the composition of the invention are also effective to combat plaque and caries.

The present invention will further be illustrated by way of Example.

### Example I

PAP, KSPB and acetylated sodium perborate with and without Imine Quat 200 were evaluated as to their bleaching efficacy.

The bleaching agents were evaluated as follows:
(1) Synthetic hydroxyapatite discs were polished and placed in sterile saliva at 37°C overnight to form a pellicle.
(2) Discs were stained with tea/coffee/iron salts/saliva mixture for seven days at 37°C.
(3) Stained discs were immersed in bleaching solutions for desired time.
(4) The change in colour of the discs was measured using a Minolta chromameter CR-300 in L*a*b* mode. Using L* (initial), L* (soiled), and L* (cleaned), the percentage of stain removed was calculated.

The concentration of the PAP and the KSPB was 3.6 x 10⁻²M in aqueous solution which also contained 0.5M sodium bicarbonate.

The amount of Imine Quat 200 was varied as indicated in the Table. The following results were obtained:
1) Percent stain removed with PAP (3.6 x 10⁻²M) in 0.5M NaHCO₃ with different concentrations of IQ200

| **% stain removed with** | | | | |
|---|---|---|---|---|
| **[IQ200] (M)** | **0 min** | **1 min** | **3 min** | **5 min** |
| 3.6 x 10⁻³ | 0 | 67 | 86 | 91 |
| 1.8 x 10⁻³ | 0 | 62 | 83 | 89 |
| 0.9 x 10⁻³ | 0 | 58 | 71 | 89 |
| 3.6 x 10⁻⁴ | 0 | 46 | 59 | 68 |
| 1.8 x 10⁻⁴ | 0 | 40 | 69 | 76 |
| 0 | 0 | 24 | 55 | 67 |

2) Percent stain removed with potassium sulphoperbenzoic acid (KSPB)(3.6 x 10⁻²M)

| **% stain removed with** | | | |
|---|---|---|---|
| **Time (mins)** | **KSPB** | **KSPB/ IQ200** | **NaHCO**_{**3**} |
| 0 | 0 | 0 | 0 |
| 0.5 | 22 | 42 | 3 |
| 1 | 34 | 55 | 3 |
| 3 | 54 | 73 | 3 |

Percent stain removed with acetylated sodium perborate (0.15 % w/w) in 0.5 M sodium bicarbonate solution with IQ200 (0.1 % w/w).

| **% stain removed with** | | |
|---|---|---|
| **Time (secs)** | **Acetylated sodium perborate** | **Acetylated sodium perborate and IQ200** |
| 0 | 0 | 0 |
| 30 | 3 | 4 |
| 60 | 16 | 22 |
| 120 | 24 | 38 |

### Example II

Bovine enamel blocks (5 x 5 x 2mm) were attached to partial or full dentures. These were worn in the mouth for 21 days in order to build up naturally stained pellicle. The blocks were removed from the dentures and treated for 15 x 1 minute with one of the following mixtures:
A - 0.5 M NaHCO₃
B - 1% PAP/0.5M NaHCO₃
C - 1% PAP + 0.11% Imine Quat 200/0.5M NaHCO₃

The colour of the bovine blocks were measured using a Minolta Chromameter CR241 in the CIE L*a*b* mode. Colour measurements were made before any treatment and after 1, 3, 5, 10 and 15 one minute treatments.

### RESULTS

These are expressed in change in L* and as % stain removed.

| **Treatment** | **Change in L*** | | | | |
|---|---|---|---|---|---|
| | **No. of 1 min treatments** | | | | |
| | **1** | **3** | **5** | **10** | **15** |
| **A** | 0.2 | 0.5 | 0.8 | 0.9 | 0.8 |
| **B** | 0.1 | 0.5 | 1.1 | 2.5 | 3.3 |
| **C** | 1.6 | 4.7 | 6.9 | 7.6 | 8.8 |
| | | | | | |

| **% stain removed** | | | | | |
|---|---|---|---|---|---|
| | **1** | **3** | **5** | **10** | **15** |
| **A** | -2 | 3 | 5 | 3 | 7 |
| **B** | 1 | 4 | 8 | 19 | 26 |
| **C** | 10 | 31 | 44 | 51 | 58 |

These results illustrate that Imine Quat catalysed peracid bleaching gives a rapid increase in L* - i.e. toothwhitening benefits after only a very short exposure.

### Example III

Example I was repeated, using different imine quaternary salts as described below. The following results were obtained:

The imine quat was modified by extending the methyl chain to butyl, hexyl and octyl, i.e. 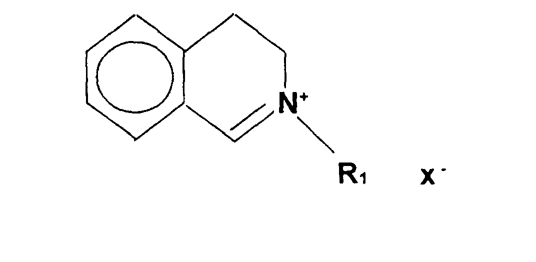 IQ200, R = methyl
IQ204, R = n-butyl
IQ206, R = n-hexyl
IQ208, R = n-octyl
X⁻ being tosylate⁻
and was tested in the in vitro stain model as described in Example I.
[PAP] = 3.6 x 10⁻²M, [IQ] = 3.6 x 10⁻³M in 0.5M sodium bicarbonate solution.

| | **% stain removed** | |
|---|---|---|
| | **1 min** | **3 mins** |
| **PAP/IQ200** | 43 | 56 |
| **PAP/IQ204** | 29 | 41 |
| **PAP/IQ206** | 23 | 36 |
| **PAP/IQ208** | 21 | 34 |
| **PAP** | 21 | 29 |

Repeating the experiment with PAP IQ206 gave the following results

| **% stain removed with** | | |
|---|---|---|
| **Time (mins)** | **PAP (s.d.)** | **PAP/IQ206 (s.d.)** |
| 0 | 0 | 0 |
| 0.5 | 9 (6) | 26 (12) |
| 1.0 | 34 (5) | 48 (9) |
| 2.0 | 44 (6) | 64 (6) |

The pH profile of IQ200 catalysts was investigated, using same concentrations as above in various phosphate and borate buffers.

| **% stain removed with** | | |
|---|---|---|
| **PH** | **PAP** | **PAP/IQ206** |
| 6.0 | 16 | 34 |
| 7.0 | 25 | 72 |
| 8.0 | 46 | 74 |
| 9.0 | 49 | 69 |

The pH profile of IQ208 was also investigated.

| **% stain removed with** | | |
|---|---|---|
| **pH** | **PAP** | **PAP/IQ208** |
| 6.0 | 32 | 78 |
| 7.0 | 49 | 76 |
| 8.0 | 45 | 59 |
| 9.0 | 72 | 71 |

The ketone and 3-methyl, 3-phenyl derivatives were also evaluated under same concentrations as above.

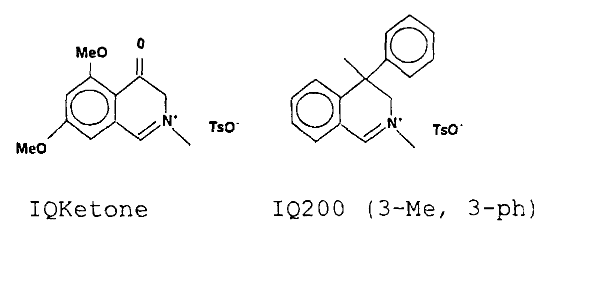

| | **% stain removed (s.d.)** | | |
|---|---|---|---|
| | **0.5 min** | **1.0 mins** | **3.0 mins** |
| PAP | 3 (3) | 23 (3) | 50 (4) |
| PAP/IQ200 | 40 (5) | 68 (2) | 84 (3) |
| PAP/IQKetone | 16 (4) | 37 (5) | 69 (3) |
| PAP/IQ200(3-Me, 3-pH) | 17 (4) | 42 (6) | 67 (3) |

IQ200-(5,7-dimethoxy-1-methyl) gives similar results.

### Example IV

Example I was repeated using magnesium monoperoxyphthalate (H48) (3.6 x 10-2M) with IQ200 (3.6 x 10-3M)

| | **% stain removed** | |
|---|---|---|
| **Time (mins)** | **H48 (s.d.)** | **H48 + IQ200 (s.d.)** |
| 0 | 0 | 0 |
| 0.5 | 27 (5) | 89 (4) |
| 1.0 | 62 (5) | 97 (2) |
| 2.0 | 80 (6) | -- |

### Example V

Bovine enamel slabs were stained with Stookey type stain and brushed with a conventional toothpaste (RDA = 100), with or without PAP/IQ200 (1 %/0.11 % w/w) in a brushing machine for 2,100 strokes. The increase in L* was measured with a Minolta CR241 Chromameter in the CIE L*a*b* mode hence the increase in whiteness determined.

| | Δ L* (s.d.) |
|---|---|
| Paste (RDA = 100) | 18.3 (3.4) |
| Paste (RDA = 100) + PAP IQ200 | 23.1 (5.3) |
| | |
| | p < 0.05 |

## Claims

1. An oral composition with an improved teeth whitening effect, comprising a safe and effective amount of
a) organic peroxy compound, selected from the group consisting of:
1) peroxy amido phthalamides having the structural formula: 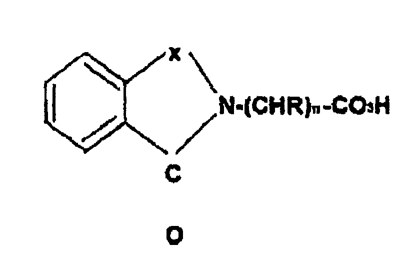 in which R = hydrogen or C₁-C₄ alkyl;
n = 1 to 5; and
X = C=0 or SO₂
2) sulphoperbenzoic acid
3) monoperoxyphthalic acid,
b) per) acetylperoxyboric acid, and/or salts thereof, **characterised in that** the composition further comprises an imine quaternary salt of the formula 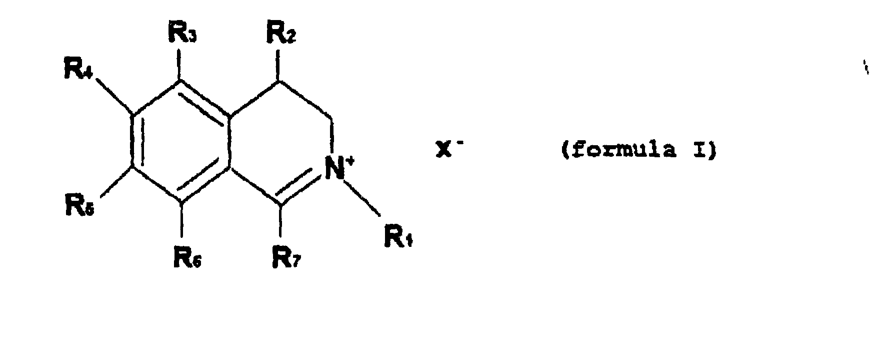
wherein R₁ is hydrogen or a C₁-C₈ alkyl group, R₂ is hydrogen, or a phenyl group or a keto group, R₃, R₄ and R₅ are hydrogen or -O-R¹, whereby R¹ is a C₁-C₄ alkyl group, R₆ and R₇ are hydrogen or a C₁-C₄ alkyl group and X is a counterion, stable in the presence of oxidizing agents, comprising Br⁻, BF₄⁻, Cl⁻, CH₃SO₄⁻, tosylate⁻, PF₆⁻, F⁻, fluorophosphate cations and C₁₂-alkylsulphate cations; and a humectant.

2. A composition according to claim 1, **characterised in that** the organic peroxy compound is N-phthalimido hexanoic peroxy acid or sulphoperbenzoic acid.

3. A composition according to claim 1 or 2, **characterised in that** the imine quaternary salt is a compound according to formula (I) wherein R₁ is methyl, R₂-R₆ are all hydrogen and X is tosylate.

4. Use of the peroxy compounds together with the imine quaternary salts of claim 1 as teeth whitening agent in the manufacture of an oral composition having improved teeth whitening properties.

## Patentansprüche

1. Orale Zusammensetzung mit verbessertem Zahnaufhellungseffekt, umfassend eine sichere und wirksame Menge einer
a) organischen Peroxyverbindung, ausgewählt aus der Gruppe, bestehend aus:
1) Peroxyamidophthalamiden mit der Strukturformel 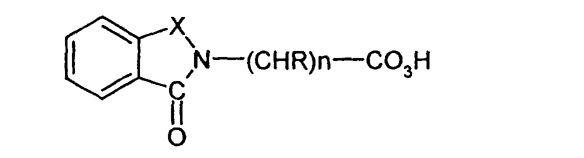 worin R = Wasserstoff oder C₁-C₄-Alkyl; n = 1 bis 5; und X = C=O oder SO₂,
2) Sulfoperbenzoesäure
3) Monoperoxyphthalsäure,
b) Per)acetylperoxyborsäure und/oder Salzen davon, **dadurch gekennzeichnet, dass** die Zusammensetzung weiterhin ein quaternäres Iminsalz der Formel 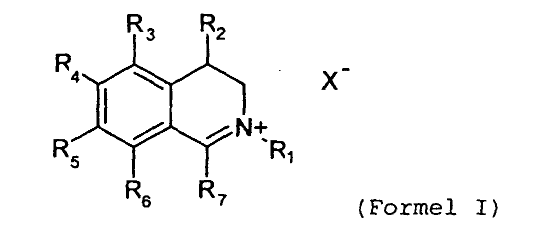
umfasst,
worin R₁ Wasserstoff oder eine C₁-C₈-Alkylgruppe darstellt, R₂ Wasserstoff oder eine Phenylgruppe oder eine Ketogruppe darstellt, R₃, R₄ und R₅ Wasserstoff oder -O-R¹ darstellen, wobei R¹ eine C₁-C₄-Alkylgruppe darstellt, R₆ und R₇ Wasserstoff oder eine C₁-C₄-Alkylgruppe darstellen und X ein Gegenion darstellt, das in Gegenwart von Oxidationsmitteln stabil ist, umfassend Br⁻, BF₄⁻, Cl⁻, CH₃SO₄⁻, Tosylat⁻, PF₆⁻, F⁻, Fluorophosphatkationen und C₁₂-Alkylsulfatkationen und ein Feuchthaltemittel.

2. Zusammensetzung nach Anspruch 1, **dadurch gekennzeichnet, dass** die organische Peroxyverbindung N-Phthalimidohexanperoxysäure oder Sulfoperbenzoesäure ist.

3. Zusammensetzung nach Anspruch 1 oder 2, **dadurch gekennzeichnet, dass** das quaternäre Iminsalz eine Verbindung der Formel (I) ist, worin R₁ Methyl darstellt, R₂-R₆ alle Wasserstoff darstellen und X Tosylat darstellt.

4. Verwendung der Peroxyverbindungen zusammen mit den quaternären Iminsalzen von Anspruch 1 als Zahnaufhellungsmittel bei der Herstellung einer oralen Zusammensetzung mit verbesserten Zahnaufhellungseigenschaften.

## Revendications

1. Composition orale avec un effet amélioré de blanchiment des dents, comprenant une quantité sûre et efficace de :
a) un composé peroxy organique, choisi dans le groupe constitué de :
1) peroxy amido phtalamides ayant la formule structurale : 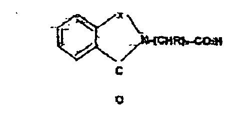 dans laquelle
R = un hydrogène ou un alkyle en C₁ à C₄ ;
n = 1 à 5 ; et
X = C=O ou SO₂
2) un acide sulphoperbenzoïque
3) un acide monoperoxyphtalique,
b) un acide peracétylperoxyborique, et/ou leurs sels,
**caractérisé en ce que** la composition comprend en outre un sel quaternaire imine de formule : 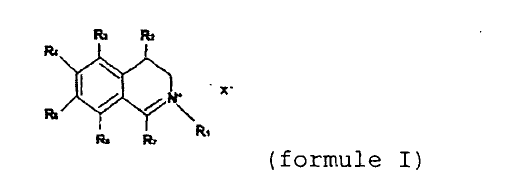 dans laquelle R₁ est un hydrogène ou un groupe alkyle en C₁ à C₈, R₂ est un hydrogène, ou un groupe phényle ou un groupe céto, R₃, R₄ et R₅ sont hydrogène ou-O-R¹, dans lequel R¹ est un groupe alkyle en C₁ à C₄, R₆ et R₇ sont hydrogène ou un groupe alkyle en C₁ à C₄ et X est un contre-ion, stable en présence d'agents oxydants comprenant Br⁻, BF₄⁻, Cl⁻CH₃SO₄⁻, tosylate⁻, PF₆⁻, F⁻, les cations fluorophosphates et les cations alkylsulphates en C₁₂ ; et un humectant.

2. Composition selon la revendication 1, **caractérisée en ce que** le composé peroxy organique est un acide N-phtalimido hexanoïque peroxy ou un acide sulphoperbenzoïque.

3. Composition selon la revendication 1 ou 2, **caractérisée en ce que** le sel quaternaire imine est un composé selon la formule (I) dans laquelle R₁ est un méthyle, R₂-R₆ sont tous hydrogène et X est un tosylate.

4. Utilisation de composés peroxy en combinaison avec les sels quaternaires imine de la revendication 1 comme agent de blanchiment des dents dans la fabrication d'une composition orale ayant des propriétés améliorées de blanchiment des dents.
